# EUROPÄISCHE PATENTSCHRIFT

(11) **EP 0 475 358 B1**
(45) Veröffentlichungstag und Bekanntmachung des Hinweises auf die Patenterteilung: **14.12.1994**
(21) Anmeldenummer: 91115288.2
(22) Anmeldetag: 10.09.1991
(51) Int. Cl.: A61F 2/30, A61C 8/00

(54) **Implantierbare prothese**
Implantable prosthesis
Prothèse implantable

(30) Priorität: 13.09.1990 DE 9013067 U; 04.01.1991 DE 9100075 U
(43) Veröffentlichungstag der Anmeldung: 18.03.1992
(73) Patentinhaber: THERA Patent GmbH & Co. KG Gesellschaft für industrielle Schutzrechte, D-82229 Seefeld (DE)
(72) Erfinder: Schmitz, Hermann-Joseph, Dr. Dr., W-1000 Berlin 41 (DE); Herold, Wolf Dietrich, Dr., W-8031 Seefeld 2 (DE); Zöllner, Werner, Dr., W-8031 Steinebach-Wörthsee (DE)
(74) Vertreter: Strehl Schübel-Hopf Groening & Partner

(56) Entgegenhaltungen:
- EP-A- 0 162 604
- EP-A- 0 377 068
- DE-A- 2 819 178
- FR-A- 2 352 536
- FR-A- 2 433 336
- GB-A- 2 181 354

## Beschreibung

Die Erfindung betrifft eine zementfrei implantierbare Prothese nach dem Oberbegriff des Anspruchs 1, wie sie aus der FR-A-2 433 336 als bekannt hervorgeht. Solche Prothesen eignen sich für den Knochen- oder Zahnwurzelersatz, als Gelenkersatz oder beispielsweise auch als starre oder flexible Bandscheibenimplantate.

Bei zementfrei implantierten Prothesen werden schädliche Nebenreaktionen der polymerisierenden Zementbestandteile im Organismus vermieden, die zur Gewebezerstörung und damit zu einer Lockerung des Implantats führen können.

Bekannte zementfrei implantierbare Prothesen sind, wie in Chemie-Ing.-Techn. 47 (1975) Nr. 8, Seiten 327 ff oder in DE-A-2 721 686 beschrieben, meist mit einer porösen Oberfläche versehen, die eine feste mechanische Verbindung mit dem Knochengewebe eingeht. Gemäß DE-A-2 154 272 kann die poröse Struktur durch Aufsintern von Kugeln oder Körnern oder durch Bohren des Prothesenschafts mittels Elektronen- oder Laserstrahls gewonnen werden.

EP-A-0 321 389 beschreibt eine Prothesen-Beschichtung aus bioaktiven, teils resorbierbaren keramischen Werkstoffen auf der Basis von Calciumphosphaten sowie aus polymeren Kunststoffen, die im Körpermilieu stabil sind. Bei der Resorption des keramischen Anteils der Prothesenverankerung bleibt ein poröses Gefüge aus Kunststoff zurück, in das Knochengewebe hineinwächst.

Bei den bekannten Prothesenverankerungen vergeht eine erhebliche Zeit, bis das Knochengewebe in die poröse Oberfläche hineingewachsen ist. In dieser Zeit ist die Belastbarkeit der Prothese gering, und es besteht die Gefahr, daß sie sich wieder lockert.

Der Erfindung liegt die Aufgabe zugrunde, eine Prothese der eingangs genannten Art zu schaffen, die auf einfache Weise implantierbar ist und rasch eine hochbelastbare, dauerhafte Verbindung mit dem Knochengewebe gewährleistet.

Diese Aufgabe wird durch die im Anspruch 1 gekennzeichnete Erfindung gelöst. Demnach ist auf der Schaftoberfläche der Prothese eine große Anzahl sacklochartiger bzw. fingerförmiger Vertiefungen angeordnet, die von einem Wulst umgeben sind. In Mikroporositäten dieser Form wächst der Knochen relativ schnell hinein. Die Wulste bewirken, vor allem wenn sie Hinterschneidungen aufweisen, schon bald nach der Implantation eine besonders sichere Verankerung der Prothese im Knochengewebe. Die feinen Sacklöcher können direkt auf der Schaftoberfläche angebracht werden, ohne daß eine als Prothesenverankerung dienende Beschichtung notwendig ist. Damit wird eine Ablösung der Prothesenverankerung, wie sie bei Beschichtungen vorkommen kann, vermieden.

Vorteilhafte Ausgestaltungen der Erfindung sind in den Unteransprüchen angegeben.

Ein Ausrichten der feinen Sacklöcher wie in Anspruch 4 angegeben, erlaubt es, gezielt gerichtete Druck- bzw. Zugkräfte von der Prothesenoberfläche in den in die Vertiefungen hineinwachsenden Knochen einzuleiten. So wird eine physiologische Belastung des Implantatbettes erzielt und eine Prothesenlockerung aufgrund Schädigung des eingewachsenen Knochengewebes vermieden.

Nach dem Transformationsgesetz der Knochen von Wolff hängen Strukturveränderungen des Knochens von seiner Belastung ab. Dort, wo der Knochen auf Druck belastet wird, kommt es zu einer Resorption, eine Zugbelastung dagegen führt zum Nachwachsen des Knochens. Durch eine entsprechende Ausrichtung der feinen Sacklöcher kann erreicht werden, daß die in die Vertiefungen hineinwachsenden Zapfen des Knochens auf Zug belastet und damit zum Wachstum angeregt werden. Dann bildet die zum Lochgrund weisende Axialrichtung mit der bei normaler Belastung von der Prothese auf den Knochen ausgeübten Kraft einen spitzen Winkel.

An bestimmten Stellen kann die Ausrichtung der Sacklöcher auch so gewählt sein, daß die Knochenzapfen gezielt auf Druck belastet werden.

Mit den in den Ansprüchen 2 und 3 angegebenen Merkmalen ergibt sich schon bald nach dem Einsetzen der Prothese eine besonders feste Verbindung mit dem Knochen. Es sind ca. 20 bis 100, vorzugsweise ca. 30 bis 50 Löcher pro mm² Schaftoberfläche mit einem Durchmesser von ca. 25 um bis 100 um, vorzugsweise ca. 30 um bis 60 um und einer Tiefe von ca. 50 um bis 200 um, vorzugsweise bis ca. 90 um vorgesehen.

Die Ausbildung der schaftoberfläche als Rundgewinde gemäß Anspruch 5 ermöglicht eine schon bald nach der Implantierung belastbare, kraft- und formschlüssige Verbindung der Prothesenverankerung mit dem Knochen. Dazu trägt neben dem mechanischen Effekt der Verschraubung auch die im Vergleich zu einem zylindrischen Schaft vergrößerte Oberfläche bei. Ein Rundgewinde weist keine scharfen Kanten auf, die zu Reizungen oder Überbeanspruchungen des Knochengewebes führen könnten.

Eine einfache Implantierbarkeit und eine dauerhafte Haltbarkeit der Prothese wird erreicht, wenn sich gemäß Anspruch 6 der Schaft vom Prothesenkopf zum Ende hin verjüngt.

Eine Titanlegierung nach Anspruch 7 ist wegen ihrer Stabilität und Bioverträglichkeit als Prothesenmaterial besonders geeignet.

Zweckmäßigerweise werden die Löcher gemäß Anspruch 8 mittels energiereicher Strahlung, beispielsweise durch Bohren mit einem Laserstrahl hergestellt. Ein Excimer-Laser ist besonders geeignet.

Bevorzugte Ausführungsbeispiele der Erfindung sind in der Zeichnung dargestellt. Es zeigen:
Figur 1 eine als Zahnimplantat gestaltete Prothese,
Figur 2 zwei Vertiefungen auf der Schaftoberfläche der Prothese im Querschnitt, und
Figur 3 eine schematische Darstellung der auf die Prothese wirkenden Kräfte.

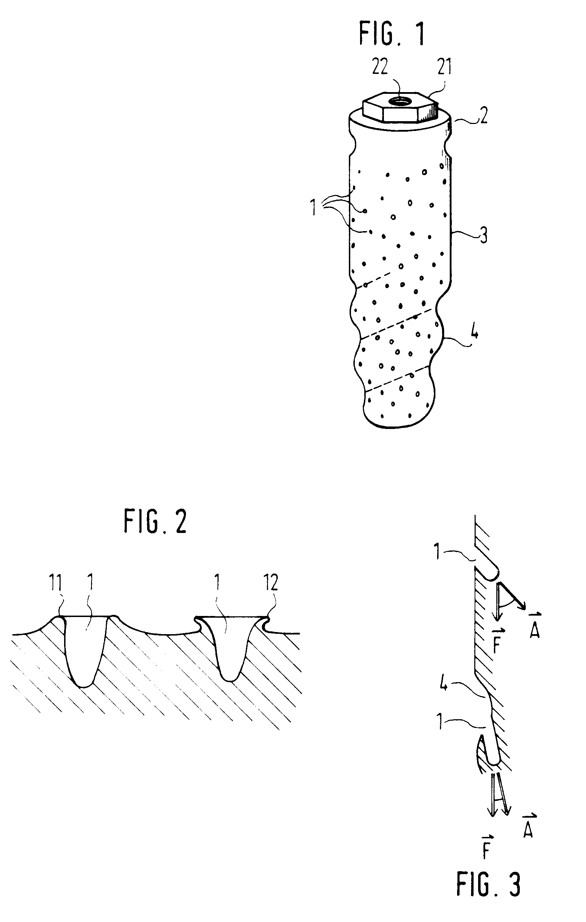

Die in Figur 1 abgebildete, als Zahnimplantat gestaltete Prothese besteht aus einem Prothesenkopf 2 und einem Schaft 3. Der Schaft 3 ist teilweise mit einem Rundgewinde 4 versehen, das einerseits schon direkt nach dem Einsetzen der Prothese für eine gewisse Stabilität sorgt und andererseits eine vergrößerte Oberfläche für den Kontakt mit dem Knochen bereitstellt. Je nach Einsatzgebiet kann es auch günstiger sein, wenn sich das Gewinde 4 über den gesamten Prothesenschaft 3 erstreckt. Eventuell kann auf ein Gewinde auch ganz verzichtet werden. Für ein problemloses Implantieren der Prothese in den Kiefer kann diese so geformt sein, daß sie sich vom Prothesenkopf 2 zum Ende hin verjüngt.

Der abgebildete Prothesenkopf 2 ist mit einem Innengewinde 22 zur Befestigung einer Zahnprothese versehen. Außerdem ist eine Rotationssicherung für die aufgeschraubte Struktur vorgesehen, die als Vierkant oder, wie dargestellt, als Sechskant 21 ausgebildet sein kann.

Zur Vergrößerung der Oberfläche und zur sicheren Befestigung der Prothese kann der Prothesenschaft anstelle des Gewindes in seinem unteren Bereich mit (nicht dargestellten) Makrozapfen versehen sein.

Die eigentliche Prothesenverankerung besteht aus den fingerförmigen, sacklochartigen Vertiefungen 1. Sie können, wie dargestellt, im wesentlichen die ganze Schaftoberfläche bedecken oder je nach der gewünschten Kraftübertragung zwischen Prothese und Knochen nur an bestimmten Stellen angebracht sein.

Die Figur 2 zeigt im Querschnitt die Vertiefungen 1 mit kraterförmigem Rand oder Wulst 11, 12 einer Laser-bestrahlten Implantatoberfläche (ELCS, Excimer Laser Conditioned Surface). Der Wulst 11, 12 entsteht durch das bei der Laserstrahl-Behandlung aus dem Loch aufgeworfene Material. In Abhängigkeit von den eingestellten Laserparametern (Energie, Strahldurchmesser, Bestrahlungsdauer, etc.) kann ein einfacher Wulst 11 oder ein mit Hinterschneidungen versehener Wulst 12 hergestellt werden. Durch Einwachsen des Knochengewebes in die Vertiefungen 1 und Umwachsen der Wülste 11, 12 wird eine doppelte mechanische Verbindung zwischen der Prothesenoberfläche und dem umgebenden knöchernen Implantatlager erreicht. Eine besonders sichere Übertragung sowohl von Druck- als auch von Zugkräften wird mit einem hinterschnittenen Wulst 12 erzielt.

In Figur 3 ist der Querschnitt durch eine Prothesenverankerung dargestellt. Die Kraft F, mit der die Prothese belastet wird und die sie wiederum auf den Knochen ausübt, wirkt in diesem Beispiel parallel zur Oberfläche. Um das Hineinwachsen des Knochengewebes in die Sacklöcher 1 zu stimulieren, sind diese so gerichtet, daß die zum Lochgrund weisende Axialrichtung A mit der Kraft F einen spitzen Winkel bildet. Die in die Vertiefungen hineinwachsenden Knochenzapfen werden dadurch im wesentlich auf Zug belastet. Dies regt nach dem Gesetz von der Transformation der Knochen von Wolff ihr Wachstum an. Die untere der beiden Vertiefungen 1 in Figur 3 ist an der schräg nach oben geneigten Fläche des Gewindes 4 angebracht. Dort läßt sich auch durch stärkeres Schrägstellen des Sacklochs 1 ein noch spitzerer Winkel zwischen Axialrichtung Ä und Kraft F und damit eine Steigerung der für das Knochenwachstum wirksamen Kraftkomponente erzielen.

Dort wo der Knochen bzw. die Knochenbälkchen natürlicherweise auf Druck belastet werden, kann es auch sinnvoll sein, die feinen Löcher so anzuordnen, daß die in sie hineinwachsenden Knochenzapfen ebenfalls eine Druckbelastung aufnehmen.

### Beispiel 1

Eine Titanoberfläche der Dimensionen 3,2 mm x 6,25 mm (20 mm²) wurde mit einem XeCI-Laser mit einer Energiedichte von 16 J/cm² behandelt. Die Einwirkdauer betrug 280 ns, und je Loch wurden 25 Pulse gesetzt. Auf der Prüffläche wurden 38 x 18 = 684 Sacklöcher einer Tiefe von ca. 60 um mit 40 bis 50 um hohen kraterförmigen Randwulsten von ca. 80 um Breite erzeugt. Die Gesamttiefe vom Kraterrand betrug somit ca. 100 um.

### Beispiel 2

Mit dem XeCI-Laser wurden in die Prüffläche nach Beispiel 1 wiederum 18 x 38 Löcher gebohrt, die mit einer Energiedichte von 10 J/cm², einer Einwirkdauer von 15 ns und einer Pulszahl von 402 erzeugt wurden. Es enstanden sich leicht in die Tiefe verjüngende Sacklöcher von ca. 100 um Tiefe mit 40 bis 60 um hohen kraterförmigen Randwulsten von ca. 70 um Breite.

## Patentansprüche

1. Zementfrei implantierbare Prothese, deren Schaftoberfläche (3) mit einer großen Anzahl feiner Sacklöcher (1) versehen ist, dadurch gekennzeichnet, daß die Sacklöcher (1) auf der Schaftoberfläche (3) von in Form eines Kraterrandes nach außen ragenden Wulsten (11, 12) umgeben sind.

2. Prothese nach Anspruch 1, dadurch gekennzeichnet, daß die Löcher (1) einen Durchmesser von ca. 25 um bis ca. 100 um, vorzugsweise ca. 30 um bis ca. 60 um und eine Tiefe von ca. 50 um bis ca. 200 um, vorzugsweise ca. 50 um bis ca. 90 um haben.

3. Prothese nach Anspruch 1 oder 2, dadurch gekennzeichnet, daß ca. 20 bis ca. 100, vorzugsweise ca. 30 bis ca. 50 Löcher (1) pro mm² Schaftoberfläche vorgesehen sind.

4. Prothese nach einem der Ansprüche 1 bis 3, dadurch gekennzeichnet, daß mindestens ein Teil der Löcher (1) so orientiert ist, daß die zum Lochgrund weisende Axialrichtung (A) mit der Richtung der bei normaler Belastung von der Prothese auf den Knochen ausgeübten Kraft (F) einen spitzen Winkel bildet.

5. Prothese nach einem der Ansprüche 1 bis 4, dadurch gekennzeichnet, daß die Schaftoberfläche (3) als Rundgewinde (4) ausgebildet ist.

6. Prothese nach einem der Ansprüche 1 bis 5, dadurch gekennzeichnet, daß sich der Schaft (3) vom Prothesenkopf (2) zum Ende hin verjüngt.

7. Prothese nach einem der Ansprüche 1 bis 6, dadurch gekennzeichnet, daß der Schaft (3) aus einer Titanlegierung besteht.

8. Prothese nach einem der Ansprüche 1 bis 7, dadurch gekennzeichnet, daß die Löcher (1) durch Bohren mittels Laserstrahl, insbesondere Excimer-Laserstahl, hergestellt sind.

## Claims

1. Cementlessly implantable prosthesis having a shaft surface (3) with a large plurality of fine pocket holes (1) characterised in that said pocket holes (1) are surrounded on the shaft surface (3) by, in the shape of crater-rims, outwardly extending bulbs (11, 12).

2. The prosthesis according to claim 1, characterised in that the holes (1) have a diameter of about 25 µm to about 100 µm, preferably about 30 µm to about 60 µm, and a depth of about 50 µm to about 200 µm, preferably about 50 µm to about 90 µm.

3. The prosthesis according to claim 1 or 2, characterised in that there are provided about 20 to about 100, preferably about 30 to about 50 holes (1) per mm² of the shaft surface.

4. The prosthesis according to any of claims 1 to 3, characterised in that at least a part of the holes (1) is oriented so that the axial direction (A) which points to the base of a hole forms an acute angel with the direction of the force (F) which is exerted by the prosthesis on the bone under normal load conditions.

5. The prosthesis according to any of claims 1 to 4, characterised in that the shaft surface (3) is shaped as a rounded thread.

6. The prosthesis according to any of claims 1 to 5, characterised in that the shaft (3) is tapered from a head of the prosthesis (2) to its end.

7. The prosthesis of any of claims 1 to 6, characterised in that the shaft (3) is made of a titanium alloy.

8. The prosthesis of any of claims 1 to 7, characterised in that the holes (1) are formed by drilling with a laser beam, particularly with an excimer laser beam.

## Revendications

1. Prothèse implantable sans ciment dont la surface de tige (3) est munie d'un grand nombre de fins trous borgnes (1), caractérisée en ce que les trous borgnes (1) sont entourés sur la surface de tige (3) de bourrelets (11, 12) qui dépassent vers l'extérieur sous la forme d'un bord de cratère.

2. Prothèse selon la revendication 1, caractérisée en ce que les trous (1) présentent un diamètre d'environ 25 µm à environ 100 um, de préférence d'environ 30 µm à environ 60 um, et une profondeur d'environ 50 µm à environ 200 um, de préférence d'environ 50 µm à environ 90 µm.

3. Prothèse selon la revendication 1 ou 2, caractérisée en ce qu'environ 20 à environ 100, de préférence environ 30 à environ 50 trous (1) sont prévus par mm² de surface de tige.

4. Prothèse selon l'une des revendications 1 à 3, caractérisée en ce qu'au moins une partie des trous (1) sont orientés de telle façon que la direction axiale (A) orientée vers le fond du trou forme un angle aigu avec la direction de la force (F) exercée par la prothèse sur l'os.

5. Prothèse selon l'une des revendications 1 à 4, caractérisée en ce que la surface de tige (3) est conformée en filet rond (4).

6. Prothèse selon l'une des revendications 1 à 5, caractérisée en ce que la tige (3) se rétrécit de la tête (2) de la prothèse en direction de l'extrémité.

7. Prothèse selon l'une des revendications 1 à 6, caractérisée en ce que la tige (3) est constituée d'un alliage de titane.

8. Prothèse selon l'une des revendications 1 à 7, caractérisée en ce que les trous (1) sont réalisés par perçage au moyen d'un faisceau laser, notamment d'un faisceau d'un laser excimer.
